**Europäisches Patentamt**

**European Patent Office**

**Office européen des brevets**

(19)

(11) Veröffentlichungsnummer: **0 007 571**
**B1**

(12) # EUROPÄISCHE PATENTSCHRIFT

(45) Veröffentlichungstag der Patentschrift: **08.04.81**

(21) Anmeldenummer: **79102524.0**

(22) Anmeldetag: **18.07.79**

(51) Int. Cl.³: **C 07 C 11/16, C 07 C 7/08,
C 07 C 11/167**

(54) **Verfahren zur Gewinnung von Butadien aus einem C₄-Kohlenwasserstoff-Gemisch.**

(30) Priorität: **28.07.78 DE 2833195**

(43) Veröffentlichungstag der Anmeldung:
**06.02.80 Patentblatt 80/3**

(45) Bekanntmachung des Hinweises auf die Patenterteilung:
**08.04.81 Patentblatt 81/14**

(84) Benannte Vertragsstaaten:
**BE DE FR GB IT NL**

(56) Entgegenhaltungen:
**DE - A1 - 2 742 148
DE - A - 1 543 119
DE - B2 - 2 122 770
DE - B2 - 2 251 051**

(73) Patentinhaber: **BASF Aktiengesellschaft
Carl-Bosch-Strasse 38
D-6700 Ludwigshafen (DE)**

(72) Erfinder: **Lindner, Alfred, Dr. Chem.
Ringstrasse 22
D-6712 Bobenheim-Roxheim 1 (DE)**
Erfinder: **Volkamer, Klaus, Dr. Chem.
Heidelberger Ring 21
D-6710 Frankenthal (DE)**
Erfinder: **Wagner, Ulrich, Dr. Phys.
Knospstrasse 7
D-6703 Limburgerhof (DE)**

Courier Press, Leamington Spa, England.

Verfahren zur Gewinnung von Butadien aus einem $C_4$-Kohlenwasserstoff-Gemisch.

Die vorliegende Erfindung betrifft ein Verfahren zur Gewinnung von Butadien aus einem Butadien und geringe Mengen Sauerstoff enthaltenden $C_4$-Kohlenwasserstoffgemisch.

Es ist bekannt, Butadien aus Butadien enthaltenden $C_4$-Kohlenwasserstoffgemischen durch extraktive Destillation unter Verwendung eines selektiven Lösungsmittels zu gewinnen. Das aus der extraktiven Destillation erhaltene Butadien wird im allgemeinen anschließend noch einer konventionellen Destillation unterworfen.

Die Butadien enthaltenden $C_4$-Kohlenwasserstoffgemische können nun in manchen Fällen geringe Mengen Sauerstoff enthalten, der in der Butadienlage zu unerwünschten Polymerbildungen führen kann, wodurch die Laufzeiten der Anlagen erheblich verringert würden. Solche geringe Mengen Sauerstoff enthaltenden $C_4$-Kohlenwasserstoffgemische werden z.B. in Anlagen zur Herstellung von Butadien-Styrol-Copolymerisation nach der Polymerisation als Styrol- und Sauerstoff-enthaltender Abfallstrom erhalten, der z.B. verbrannt werden kann, jedoch zweckmäßigerweise in eine Butadienextraktionsanlage zurückgeführt wird.

Weiter können die aus Äthylenanlagen erhaltenen $C_4$-Kohlenwasserstoffgemische geringe Mengen Sauerstoff aufweisen, z.B. bei Störungen in den Äthylenanlagen oder bei unsachgemäßem Transport der $C_4$-Kohlenwasserstoffgemische.

Es ist bereits vorgeschlagen worden, den Sauerstoff aus den Sauerstoff enthaltenden $C_4$-Kohlenwasserstoffgemischen durch Abdampfen oder durch Abstreifen mit Stickstoff aus den $C_4$-Kohlenwasserstoffgemischen zu entfernen. Diese Arbeitsweise ist jedoch mit hohen Verlusten an Kohlenwasserstoffen verbunden.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Gewinnung von Butadien mit Hilfe eines selektiven Lösungsmittels aus einem $C_4$-Kohlenwasserstoffgemische, welches Butadien, geringe Mengen Sauerstoff, im selektiven Lösungsmittel löslichere Kohlenwasserstoffe als Butadien und im selektiven Lösungsmittel weniger lösliche Kohlenwasserstoffe als Butadien enthält, bei dem das $C_4$-Kohlenwasserstoffgemisch durch extraktive Destillation in ein die weniger löslichen Kohlenwasserstoffe enthaltendes Destillat einen Strom aus Butadien und einen die löslicheren Kohlenwasserstoffe enthaltenden Strom aufgetrennt wird, welches dadurch gekennzeichnet ist, daß aus dem $C_4$-Kohlenwasserstoffgemisch in einer der extraktiven Destillation vorgeschalteten Destillationszone ein Gemisch aus Sauerstoff und $C_4$-Kohlenwasserstoffen abgetrennt wird und das Sumpfprodukt der extraktiven Destillation zugeführt wird.

Nach dem neuen Verfahren ist es möglich, Butadien aus $C_4$-Kohlenwasserstoffgemischen zu gewinnen, ohne daß Sauerstoff mit den Kohlenwasserstoffen in die Anlage eingeschleppt wird, so daß eine kontinuierliche Durchführung des Verfahrens über ausgedehnte Zeiträume möglich wird.

Die für die Gewinnung von Butadien einzusetzenden $C_4$-Kohlenwasserstoffgemische werden z.B. bei der Herstellung von Athylen und/oder Propylen durch thermisches Spalten einer Petroleumfraktion, z.B. von verflüssigtem Petroleumgas (LPG), Leichtbenzin (Naphtha), Gasöl und dergleichen, als Kohlenwasserstofffraktion erhalten. Weiterhin werden solche $C_4$-Fraktionen bei der katalytischen Dehydrierung von n-Butan und/oder n-Buten erhalten. Weiter werden geeignete $C_4$-Kohlenwasserstoffgemische mit relativ hohem Butadiengehalt bei der Herstellung von Polybutadien oder Butadien-Styrol-Copolymeren erhalten. Das $C_4$-Kohlenwasserstoffgemisch enthält in der Regel Butane, n-Buten, Isobuten, Butadien, Vinylacetylen, Äthylacetylen, Butadien-1,2 und gegebenenfalls Styrol, Vinylcyclohexan, geringe Mengen $C_3$-Kohlenwasserstoffe und $C_5$-Kohlenwasserstoffe. Die $C_4$-Kohlenwasserstoffgemische können, z.B. aufgrund von Störungen in der Stufe zur Erzeugung der $C_4$-Kohlenwasserstoffgemische oder aufgrund von Undichtigkeiten oder unzureichender Stickstoffspülung der Apparate und Rohrsysteme und Behälter für den Transport sowie aufgrund der Sauerstoffinitiierung bei der Herstellung von Polybutadien oder Butadien-Styrol-Copolymerisaten geringe Mengen Sauerstoff enthalten. Im allgemeinen weisen die erfindungsgemäß einzusetzenden $C_4$-Kohlenwasserstoffgemische Sauerstoffgehalte von 0,0001 bis 1 Gewichtsprozent, meistens von 0,001 bis 0,5 Gewichtsprozent auf. Das erfindungsgemäße Verfahren ist jedoch auch auf $C_4$-Kohlenwasserstoffgemische mit höheren Sauerstoffgehalten anwendbar.

Als selektives Lösungsmittel kommen beispielsweise Carbonsäureamide, wie Dimethylformamid, Diäthylformamid, Dimethylacetamid, Formylmorpholin, Acetonitril, Furfurol, N-Methylpyrrolidon, Butylrolacton, Aceton und ihre Mischungen mit Wasser in Betracht. Mit besonderem Vorteil wird N-Methylpyrrolidon als selektives Lösungsmittel verwendet.

Im selektiven Lösungsmittel löslichere Kohlenwasserstoffe als Butadien sind beispielsweise Vinylacetylen, Äthylacetylen, Butadien-1,2. Im selektiven Lösungsmittel weniger lösliche Kohlenwasserstoffe als Butadien sind beispielsweise die Butane, die n-Butene, Isobuten.

Die extraktive Destillation kann unter Anwendung einer Extraktivdestillationszone durchgeführt werden. Mit besonderem Vorteil wird das Verfahren zur Gewinnung von Butadien jedoch unter Anwendung von zwei hintereinandergeschalteten Extraktivdestillationszonen unter Verwendung des gleichen selektiven Lösungsmittels durchgeführt. Hierbei wird beispielsweise in der ersten Stufe der Extraktivdestillation ein die weniger löslichen Kohlenwasserstoffe enthaltendes Destillat (Raffinat) und ein Butadien und die löslicheren Kohlenwasserstoffe und das selektive Lösungsmittel enthaltender Extrakt erhalten. Dieser Extrakt wird vom selektiven Lösungsmittel befreit, wobei ein Gemisch aus Butadien und den löslicheren Kohlenwasserstoffen erhalten wird. Dieses Gemisch wird in einer zweiten extraktiven Destillationszone einer zweiten extraktiven Destillation mit dem selektiven Lösungsmittel unterworfen, wobei Butadien als Destillat und ein Extrakt erhalten werden, der die löslicheren Kohlenwasserstoffe einschließlich der höheren Acetylene und noch zurückgebliebenes Butadien und das selektive Lösungsmittel enthält. Der erhaltene Extrakt wird anschließend vom selektiven Lösungsmittel befreit, wobei ein die löslicheren Kohlenwasserstoffe einschließlich der $C_4$-Acetylene enthaltender Kohlenwasserstoffstrom erhalten wird.

Das aus der extraktiven Destillation erhaltene Butadien wird im allgemeinen zur Entfernung von noch gegebenenfalls vornandenen geringen Mengen von Propin und $C_5$-Kohlenwasserstoffen einer nachgeschalteten Destillation, z.B. in einer oder zwei Destillationskolonnen, unterworfen.

Die Destillative Abtrennung von gegebenenfalls in den Ausgangs-$C_4$-Kohlenwasserstoffgemischen enthaltenem Propin und enthaltenen $C_5$-Kohlenwasserstoffen kann jedoch auch in ein oder mehreren, z.B. 2, der extraktiven Destillation vorgeschalteten Destillationskolonnen erfolgen, z.B. in Kolonnen, die zwischen der vorgeschalteten Destillationszone für die Sauerstoffabtrennung und der extraktiven Destillation zwischengeschaltet werden.

Die aus der Herstellung von Styrol-Butadien-Copolymerisaten erhaltenen sauerstoffhaltigen $C_4$-Kohlenwasserstoffgemische weisen in der Regel noch einen Gehalt an Styrol und gegebenenfalls Vinylcyclohexen auf. Auch diese $C_4$-Kohlenwasserstoffgemische werden im allgemeinen nach der erfindungsgemäßen Sauerstoffabtrennung anschließend zur Abtrennung des Styrols und gegebenenfalls des Vinylcyclohexens einer der extraktiven Destillation vorgeschalteten Destillation, z.B. in einer oder mehreren, beispielsweise 2, Destillationskolonnen unterworfen.

Erfindungsgemäß wird aus dem geringe Mengen Sauerstoff enthaltenden Ausgangs-$C_4$-Kohlenwasserstoffgemisch in einer der extraktiven Destillation vorgeschalteten Destillationszone ein Gemisch aus Sauerstoff und $C_4$-Kohlenwasserstoffen, zweckmäßig als Kopfprodukt, abgetrennt und das Sumpfprodukt der vorgeschalteten Destillationszone der extraktiven Destillation zugeführt. Im allgemeinen weist das Gemisch aus Sauerstoff und den $C_4$-Kohlenwasserstoffen einen Sauerstoff-Gehalt von 1 bis 18 Molprozent, vorzugsweise von 5 bis 16 Molprozent, insbesondere 10 bis 15 Molprozent auf, wobei sich die obere Begrenzung des Sauerstoffgehaltes aus Sicherheitsgründen ergibt.

In einer vorteilhaften Ausführungsform des erfindungsgemäßen Verfahrens wird der vorgeschalteten Destillationszone ein zweiter Strom von flüssigen oder gasförmigen Kohlenwasserstoffen zugeführt. Durch diese Arbeitsweise kann der mit der Abtrennung des Gemisches aus Sauerstoff und $C_4$-Kohlenwasserstoffen verbundene Butadien-Verlust sehr gering gehalten werden.

Als flüssige oder gasförmige Kohlenwasserstoffe, die der Destillationszone als zweiter Strom zugeführt werden, werden im allgemeinen $C_3$- und/oder $C_4$-Kohlenwasserstoffe eingesetzt. Zweckmäßigerweise werden Kohlenwasserstoffe mit einem geringeren Butadiengehalt als das Ausgangs-$C_4$-Kohlenwasserstoffgemisch, z.B. mit weniger als 90% des Butadiengehaltes des Ausgangs-$C_4$-Kohlenwasserstoffgemisches, vorzugsweise im wesentlichen Butadien-freie Kohlenwasserstoffe, z.B. mit einem Butadiengehalt von weniger als 5 Gewichtsprozent, zweckmäßig weniger als 1 Gewichtsprozent eingesetzt. Vorteilhaft werden gesättigte und/oder einfach olefinisch ungesättigte $C_3$- und/oder $C_4$-Kohlenwasserstoffe verwendet. Zweckmäßig werden diese Kohlenwasserstoffe in Form von Gemischen, z.B. als $C_3$- oder $C_4$-Kohlenwasserstoffgemische verwendet. Geeignete Kohlenwasserstoffe sind z.B. Propan, Propen, die Butane, n-Buten, Isobuten, die 2-Butene, Butadien-1,2 oder diese Kohlenwasserstoffe enthaltende Gemische. Vorteilhaft werden $C_4$-Kohlenwasserstoffgemische eingesetzt. In einer bevorzugten Ausführungsform des Verfahrens wird das in der extraktiven Destillation erhaltene Raffinat verwendet, welches die Butane und Butene enthält.

Im allgemeinen wird der der vorgeschalteten Destillationszone zuzuführende zweite Kohlenwasserstoffstrom am oder zweckmäßig oberhalb des Zulaufpunktes des geringe Mengen Sauerstoff enthaltenden Ausgangs-$C_4$-Kohlenwasserstoffgemisches der vorgeschalteten Destillationszone zugeführt, wobei eine Zuführung im oberen Drittel, vorteilhaft im oberen Viertel, insbesondere am Kopf der vorgeschalteten Destillationszone, bevorzugt wird. Durch die Zuführung des zweiten Kohlenwasserstoffstromes oberhalb des Zulaufpunktes des Ausgangs-$C_4$-Kohlenwasserstoffgemisches wird durch die dadurch erfolgende Gegenstromwäsche als Kopfprodukt ein Gemisch aus Sauerstoff und $C_4$-Kohlenwasserstoffen mit einem besonders geringen Butadiengehalt, z.B. von weniger als 10 Gewichtsprozent, vorzugsweise weniger als 5 Gewichtsprozent Butadien, abgetrennt, so daß durch diese Arbeitsweise der mit - der Sauerstoff-Abtrennung verbundene Butadienverlust besonders niedrig gehalten werden kann. In einer bevorzugten Ausführungsform des erfindungsgemäßen Verfahrens wird der zweite Kohlenwasserstoffstrom dem flüssigen Rücklauf der vorgeschalteten Destillationszone zugemischt oder anstelle eines flüssigen Rücklaufs zugemischt.

Das Ausgangs-$C_4$-Kohlenwasserstoffgemisch wird der vorgeschalteten Destillationszone zweckmäßig in einem mittleren Bereich zugeführt, der sich von der Mitte der Destillationszone ausgehend bis jeweils zu etwa 80%, vorzugsweise zu etwa 60%, insbesondere zu etwa 50% in die obere und untere Hälfte der vorgeschalteten Destillationszone erstreckt. Das Ausgangs-$C_4$-Kohlenwasserstoffgemisch und der zweite Kohlenwasserstoffstrom können gasförmig oder flüssig der weiteren Destillationszone zugeführt werden. Zweckmäßig werden sie flüssig zugeführt. Im allgemeinen beträgt das Gewichtsverhältnis der Kohlenwasserstoffe des zweiten Kohlenwasserstoffstromes zu denen des Ausgangs-$C_4$-Kohlenwasserstoffgemisches 1:1 bis 1:100 vorzugsweise 1:2 bis 1:50 insbesondere 1:5 bis 1:20. Für die vorgeschaltete Destillationszone werden im allgemeinen herkömmliche Destillationskolonnen, z.B. Füllkörperkolonnen oder Bodenkolonnen verwendet. Das Sumpfprodukt der vorgeschalteten Destillationskolonne wird der extraktiven Destillation zugeführt.

In einer weiteren Ausführungsform des erfindungsgemäßen Verfahrens wird aus dem geringe Mengen Sauerstoff enthaltenden Ausgangs-$C_4$-Kohlenwasserstoffgemisch zunächst in der extraktiven Destillation vorgeschalteten Destillationszone ein Gemisch aus Sauerstoff und $C_4$-Kohlenwasserstoffen abgetrennt und das Sumpfprodukt der vorgeschalteten Destillationszone der extraktiven Destillation zugeführt. Das aus der vorgeschalteten Destillationszone abgetrennte Germisch aus Sauerstoff und $C_4$-Kohlenwasserstoffen wird danach einer weiteren Destillationszone zugeführt, · wobei der weiteren Destillationszone gleichzeitig ein zweiter Strom von flüssigen oder gasförmigen Kohlenwasserstoffen zugeführt wird und das Sumpfprodukt der weiteren Destillationszone in den Hauptstrom zurückgeführt wird. Zweckmäßig wird das Sumpfprodukt dem Ausgangs-$C_4$-Kohlenwasserstoffgemisch zugemischt. Im allgemeinen wird der der weiteren Destillationszone zuzuführende zweite Kohlenwasserstoffstrom am oder zweckmäßig oberhalb des Zulauf-punktes des abgetrennten Gemisches aus Sauerstoff und $C_4$-Kohlenwasserstoffen der weiteren Destillationszone zugeführt, wobei eine Zuführung im oberen Drittel, vorteilhaft im oberen Viertel, insbesondere am Kopf der weiteren Destillationszone, bevorzugt wird. Vorzugsweise wird der zweite Kohlenwasserstoffstrom dem flüssigen Rücklauf der weiteren Destillationszone zugemischt oder anstelle eines flüssigen Rücklaufs zugemischt.

Das abgetrennte Gemisch aus Sauerstoff und $C_4$-Kohlenwasserstoffen wird der weiteren · Destillationszone zweckmäßig in einem mittieren Bereich zugeführt, der sich von der Mitte der Destillationszone ausgehend bis jeweils zu etwa 80%, vorzugsweise zu etwa 60%, insbesondere zu etwa 50% in die obere und untere Hälfte der weiteren Destillationszone erstreckt. Das abgetrennte Gemisch aus Sauerstoff und $C_4$-Kohlenwasserstoffen und der zweite Kohlenwasserstoffstrom können gasförmig oder flüssig der weiteren Destillationszone zugeführt werden. Zweckmäßig werden sie flüssig zugeführt. Im allgemeinen beträgt das Gewichtsverhältnis der Kohlenwasserstoffe des zweiten Kohlenwasserstoffstromes zu denen des abgetrennten Gemisches aus Sauerstoff und $C_4$-Kohlenwasserstoffes 1:1 bis 1:100, vorzugsweise 1:2 bis 1:50, insbesondere 1:5 bis 1:20. Für die weitere Destillationszone werden im allgemeinen herkömmliche Destillationskolonnen, z.B. Füllkörperkolonnen oder Bodenkolonnen verwendet.

Es kann vorteilhaft sein, zusätzlich zu der Sauerstoff-Abtrennung aus dem Ausgangs-$C_4$ Kohlenwasserstoffgemisch in der vorgeschalteten Destillationszone bei der anschließenden extraktiven Destillation in an sich bekannter Weise Polymerisationsinhibitoren zuzugeben. Geeignete Polymerisationsinhibitoren sind z.B. Furfurol, Benzaldehyd, aliphatische oder aromatische Nitroverbindungen, Hydrochinon, Schwefel, Natriumnitrit, phenolische Verbindungen, wie 4-tert.-Butylcatechol und aromatische Amine, wie Naphthylamin. Hierdurch kann die Polymerisationsneigung des Butadiens zusätzlich vermindert werden. Diese Inhibitoren können ebenso in den Destillationen zur Sauerstoffabtrennung eingesetzt werden.

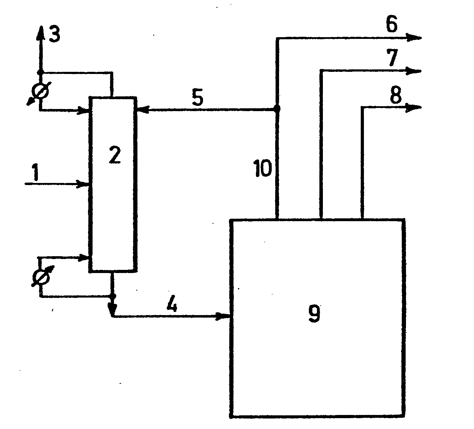

Die Figur ist ein schematisches Diagramm einer Ausführungsform des erfindungsgemäßen Verfahrens. Ein geringe Mengen Sauerstoff enthaltendes $C_4$-Kohlenwasserstoffgemisch ($C_4$-Fraktion aus einer Äthylenanlage) wird durch Leitung 1 in das mittlere Drittel einer Destillationskolonne 2 eingeführt. Am Kopf der Kolonne 2 wird durch Leitung 5 ein Teilstrom des Raffinats einer nachgeschalteten Butadien-Gewinnungsanlage 9 zugeführt. Am Sumpf der Kolonne 2 wird durch Leitung 4 ein Sauerstofffreies $C_4$-Kohlenwasserstoffgemisch abgezogen und der Butadienanlage 9 zugeführt, in der es durch extraktive Destillation unter Verwendung eines selektiven Lösungsmittels (N-Methylpyrrolidon) in eine die Butane und Butene enthaltende Fraktion (Raffinat) 10, den Butadien-Strom 7 und den die $C_4$-Acetylene enthaltenden Strom 8 aufgetrennt wird. Der nach Abzweigen des Stromes 5 verbleibende Teilstrom des Raffinats wird über Leitung 6 abgezogen.

Das nachstehende Beispiel dient der weiteren Erläuterung der Erfindung.

## Beispiel 1

Ein C_4-Kohlenwasserstoffgemisch hat die folgende Zusammensetzung:

| | |
|---|---|
| C_3-Kohlenwasserstoffe | 0,3 Gew.-% |
| n-Butan | 3,9 Gew.-% |
| Isobutan | 1,3 Gew.-% |
| N-Buten-1 | 10,8 Gew.-% |
| Isobuten | 20,9 Gew.-% |
| Buten-2-trans | 3,8 Gew.-% |
| Buten-2-cis | 3,2 Gew.-% |
| Butadien | 52,4 Gew.-% |
| Butadien-1,2 | 0,4 Gew.-% |
| Butenin | 2,0 Gew.-% |
| Butin | 0,1 Gew.-% |
| Sauerstoff | 0,3 Gew.-% |

1000 g/h dieses C_4-Kohlenwasserstoffgemisches werden am 5. Boden einer Kolonne mit 10 Böden eingeleitet. Am Kopf der Kolonne werden 100 g/h Raffinat aus einer nachgeschalteten Butadien-Gewinnungsanlage der folgenden Zusammensetzung zugeführt:

| | |
|---|---|
| C_3-Kohlenwasserstoffe | 0,4 Gew.-% |
| Butane | 11,7 Gew.-% |
| Butene | 87,8 Gew.-% |
| Butadien | 0,1 Gew.-% |

Am Sumpf der Kolonne werden 1070 g/h eines praktisch Sauerstoff-freien (<0,5 Gew.ppm $O_2$) C_4-Kohlenwasserstoffgemisches abgezogen und der Butadien-Gewinnungsanlage zugeführt, in der es durch extraktive Destillation in zwei hintereinandergeschalteten extraktiven Destillationszonen unter Verwendung von N-Methylpyrrolidon als selektives Lösungsmittel und Natriumnitrit als Polymerisationsinhibitor in ein die Butane und Butene enthaltendes Destillat, hochreines Butadien und einen die C_4-Acetylene enthaltenden Strom aufgetrennt wird. Das Kopfprodukt der Kolonne enthält 3,3 Gewichtsprozent Butadien, entsprechend einem Butadienverlust von 0,2%.

## Beispiel 2

Man verfährt wie in Beispiel 1 beschrieben, wobei jedoch kein Raffinat zugeführt wird. Der Butadienverlust im Kopfprodukt beträgt 5,8%.

## Patentansprüche

1. Verfahren zur Gewinnung von Butadien mit Hilfe eines selektiven Lösungsmittels aus einem C_4-Kohlenwasserstoffgemisch, welches Butadien, geringe Mengen Sauerstoff, im selektiven Lösungsmittel löslichere Kohlenwasserstoffe als Butadien und im selektiven Lösungsmittel weniger lösliche Kohlenwasserstoffe als Butadien enthält, bei dem das C_4-Kohlenwasserstoffgemisch durch extraktive Destillation in ein die weniger löslichen Kohlenwasserstoffe enthaltendes Destillat, einen Strom aus Butadien und einen die löslicheren Kohlenwasserstoffe enthaltenden Strom aufgetrennt wird, dadurch gekennzeichnet, daß aus dem C_4-Kohlenwasserstoffgemisch in einer der extraktiven Destillation vorgeschalteten Destillationszone ein Gemisch aus Sauerstoff und C_4-Kohlenwasserstoffen abgetrennt wird und das Sumpfprodukt der extraktiven Destillation zugeführt wird.

2. Verfahren nach Anspruch 1, dadurch gekennzeichnet, daß der vorgeschalteten Destillationszone gleichzeitig ein zweiter Strom von flüssigen oder gasförmigen Kohlenwasserstoffen zugeführt wird.

3. Verfahren nach Anspruch 1, dadurch gekennzeichnet, daß das abgetrennte Gemisch aus Sauerstoff und C_4-Kohlenwasserstoffen einer weiteren Destillationszone zugeführt wird, wobei der weiteren Destillationszone gleichzeitig ein zweiter Strom von flüssigen oder gasförmigen Kohlenwasserstoffen zugeführt wird und das Sumpfprodukt der weiteren Destillationszone in den Hauptstrom zurückgeführt wird.

4. Verfahren nach Anspruch 2 und 3, dadurch gekennzeichnet, daß als zweiter Strom ein gesättigter oder einfach olefinisch ungesättigter C_4-Kohlenwasserstoff bzw. ein Gemisch aus gesättigten und/oder einfach olefinisch ungesättigten C_4-Kohlenwasserstoffen zugeführt wird.

5. Verfahren nach Anspruch 2 bis 4, dadurch gekennzeichnet, daß der zweite Kohlenwasserstoffstrom am oder oberhalb des Zulaufpunktes des Sauerstoff enthaltenden Kohlenwasserstoffstroms der betreffenden Destillationszone zugeführt wird.

6. Verfahren nach Anspruch 2 bis 4, dadurch gekennzeichnet, daß der zweite Kohlenwasserstoffstrom im oberen Drittel der betreffenden Destillationszone zugeführt wird.

7. Verfahren nach Anspruch 1 bis 6, dadurch gekennzeichnet, daß zwischen der vorgeschalteten Destillationszone und der extraktiven Destillation eine oder mehrere Destillationskolonnen zwischengeschaltet werden.

## Claims

1. A process for isolating butadiene by means of a selective solvent from a $C_4$-hydrocarbon mixture which contains butadiene, small amounts of oxygen, hydrocarbons which are more soluble than butadiene in the selective solvent and hydrocarbons which are less soluble than butadiene in the selective solvent, wherein the $C_4$-hydrocarbon mixture is separated, by extractive distillation, into a distillate containing the less soluble hydrocarbons, a stream of butadiene and a stream containing the more soluble hydrocarbons, characterized in that a mixture of oxygen and $C_4$-hydrocarbons is separated out of the $C_4$-hydrocarbon mixture in a distillation zone upstream of the extractive distillation, and the bottom product is fed to the extractive distillation.

2. A process as claimed in claim 1, characterized in that a second stream of liquid or gaseous hydrocarbons is fed simultaneously to the upstream distillation zone.

3. A process as claimed in claim 1, characterized in that the mixture of oxygen and $C_4$-hydrocarbons which has been separated out is fed to an additional distillation zone, a second stream of liquid or gaseous hydrocarbons being fed simultaneously to the additional distillation zone, and the bottom product of the additional distillation zone being recycled to the mains stream.

4. A process as claimed in claims 2 and 3, characterized in that a saturated or monoolefinically unsaturated $C_4$-hydrocarbon, or a mixture of saturated and/or monoolefinically unsaturated $C_4$-hydrocarbons is fed in as the second stream.

5. A process as claimed in claims 2 to 4, characterized in that the second hydrocarbon stream is fed in at or above the feed point of the oxygen-containing hydrocarbon stream of the particular distillation zone.

6. A process as claimed in claims 2 to 4, characterized in that the second hydrocarbon stream is fed in in the upper third of the particular distillation zone.

7. A process as claimed in claims 1 to 6, characterized in that one or more distillation collumns are interposed between the upstream distillation zone and the extractive distillation.

## Revendications

1. Procédé pour la récupération de butadiène à l'aide d'un solvant sélectif à partir d'un mélange d'hydrocarbures à 4 C qui contient du butadiène, de petites quantités d'oxygène, des l'hydrocarbures plus solubles que le butadiène dans le solvant sélectif et des hydrocarbures moins solubles que le butadiène das le solvant sélectif, le mélange d'hydrocarbures à 4 C étant séparé par distillation extractive en un distillat qui contient les hydrocarbures moins solubles, en un courant de butadiène et en un courant contenant les hydrocarbures plus solubles, caractérisé en ce qu'un mélange d'oxygene et d'hydrocarbures à 4 C est séparé du mélange d'hydrocarbures à 4 C dans une zone de distillation intercalée en amont de la distillation extractive et en ce que le produit de bas de colonne est dirigé vers la distillation extractive.

2. Procédé selon la revendication 1, caractérisé en ce qu'il est introduit en même temps, dans la zone de distillation intercalée en amont, un second courant d'hydrocarbures liquides ou gazeux.

3. Procédé selon la revendication 1, caractérisé en ce que le mélange séparé d'oxygène et d'hydrocarbures à 4 C est dirigé vers une zone de distillation supplémentaire, un second courant d'hydrocarbures liquides ou gazeux étant introduit en même temps dans la zone de distillation supplémentaire et le produit de bas de colonne de la zone de distillation supplémentaire étant ramené dans le courant principal.

4. Procédé selon la revendication 2 ou 3, caractérisé en ce qu'il est introduit, en tant que second courant, un hydrocarbure à 4 C saturé ou présentant une simple insaturation oléfinique ou un mélange d'hydrocarbures à 4 C insaturés et/ou présentant une simple insaturation oléfinique.

5. Procédé selon l'une quelconque des revendications 2 à 4, caractérisé en ce que le second courant d'hydrocarbures est introduit dans la zone de distillation en question au niveau ou au-dessus du point d'admission du courant d'hydrocarbures contenant de l'oxygène.

6. Procédé selon l'une quelconque des revendications 2 à 4, caractérisé en ce que le second courant d'hydrocarbures est introduit dans le tiers supérieur de la zone de distillation en question.

7. Procédé selon l'une quelconque des revendications 1 à 6, caractérisé en ce qu'une ou plusieurs colonnes de distillation sont interposées entre la zone de distillation intercalée en amont et la distillation extractive.